# FASCICULE DE BREVET EUROPEEN

(11) **EP 3 430 087 B1**
(45) Date de publication et mention de la délivrance du brevet: **01.01.2020**
(21) Numéro de dépôt: 17715514.0
(22) Date de dépôt: 16.03.2017
(51) Int. Cl.: C08L 83/04, C09J 183/04, A61L 27/18, A61F 2/12, A61L 26/00

(54) **GEL SILICONE ADHÉSIF À LA PEAU**
AUF HAUT HAFTENDES SILIKONGEL
SKIN-ADHESIVE SILICONE GEL

(30) Priorité: 17.03.2016 FR 1600444
(43) Date de publication de la demande: 23.01.2019
(73) Titulaire: ELKEM SILICONES France SAS, 69003 Lyon (FR)
(72) Inventeur: MOINE, Caroline, 42290 Sorbiers (FR); QUEMIN, Maryline, 69100 Villeurbanne (FR); CROS, Gaelle, 69360 Ternay (FR)
(74) Mandataire: Mekki, Boualem
(86) Numéro de dépôt international: PCT/FR2017/000051
(87) Numéro de publication internationale: WO 2017/158250

(56) Documents cités:
- WO-A1-2008/057155

## Description

La présente invention concerne un gel silicone utile dans les prothèses mammaires, les coussins et matelas de prévention des escarres ou pour une utilisation contre la peau et plus particulièrement;
- dans un pansement adhésif à la peau, ou partie d'un tel pansement, en particulier destinés au retrait atraumatique sur une peau saine ou sur une plaie, ou
- dans un dispositif de maintien d'accessoires médicaux tel qu'un dispositif de fixation de sacs de stomie, ou partie d'un tel dispositif utilisés au contact de la peau de type capteur, sonde, cathéter ou aiguille.

Les gels silicones sont traditionnellement obtenus par réticulation d'une composition comprenant un organopolysiloxane porteur d'au moins deux fonctions vinyles par molécule, un polyorganohydrogénosiloxane porteur d'au moins trois fonctions SiH par molécule (appelé « réticulant » ou « crosslinker »), d'un di-(hydrogénosilyl)organopolysiloxane porteur de deux fonctions SiH (appelé « allongeur » ou « chain extender ») et un catalyseur d'hydrosylilation à base de platine. Ces gels silicones sont traditionnellement utilisés pour la protection de matériels électroniques sensibles aux vibrations, aux chocs, à la température et de manière plus générale aux agressions physiques et chimiques de l'atmosphère ambiante. Pour leur mise en œuvre dans cette application, les gels silicones encapsulent les composants électroniques ("Potting"). Les gels silicones sont aussi utilisés comme matériau médical de base, notamment pour l'élaboration de prothèse mammaire ou de pansements. Ils sont aussi utilisés comme adhésif, pour autant que leurs propriétés adhérentes soient significatives et comme matériau amortisseur de choc. Pour toutes ces applications, les propriétés physiques de ces gels sont adaptées suivant l'utilisation en faisant varier les taux de motifs siloxyles porteurs de fonctions Si-alcényle (le plus souvent des fonctions Si-vinyles) et SiH.

C'est ainsi que la majorité des compositions silicones précurseurs de gels silicones qui sont actuellement commercialisées sont préparés à partir de composition silicones réticulant par des réactions de polyadditions dans lesquelles le rapport molaire RHAlk = tH/tAlk ≤ 1,0 et le plus souvent proche de 0,8 avec :
- tH = nombre de moles d'atome d'hydrogène directement lié à un atome de silicium des organopolysiloxanes porteurs de fonctions réactives SiH,
- tAlk = nombre de mole d'alcényle directement lié à un atome de silicium des organopolysiloxanes porteurs de fonctions réactives alcénylées,

La référence WO-2008/057155 décrit des compositions formant un gel de silicone qui ont un RHAlk moyen de 0,7 à 1,5, typiquement de 0,8 à 0,95 et avec un RHCE moyen de 0,4 à 1, typiquement 0,8 à 0,95. RHCE = (moles d'hydrogène lié au silicium à partir d'allongeurs de chaîne) / (moles d'hydrogène lié au silicium). Ces gels sont adaptés pour adhérer temporairement un dispositif médical à un substrat biologique tel que la peau. Les gels de silicone fournissent une adhérence élevée à la peau et une faible force de libération de la peau du substrat de polyéthylène.

Cependant, la référence EP-322118 décrit des compositions silicones précurseurs de gels silicone ayant une cinétique de réticulation rapide dans lesquelles le rapport molaire RHAlk varie de 1 à 20. Ces compositions silicones réticulant par hydrosilylation se caractérisent par un excès en fonctions réactives SiH vis-à-vis des fonctions Si-alcényles. Les compositions silicones décrites sont limitées aux compositions comprenant :
- un organopolysiloxane ayant au moins 3 fonctions SiH (jouant le rôle de réticulant),
- un organopolysiloxane ayant des terminaisons diorganohydrogénosiloxy (= un « allongeur de chaine » ayant 2 fonctions SiH),
- un organopolysiloxane ayant deux fonctions Si-alcényles par polymère et dont la viscosité cinématique à 25°C est comprise entre 50 à 10000 mm².s.

Les exemples mettent en œuvre des compositions silicones ayant des RHAlk de 3, 4, 7 et 10 avec des polymères réticulants qui contiennent 0,7% en poids d'atome d'hydrogène provenant de fonctions SiH, ou 1,5 % en poids d'atome d'hydrogène provenant de fonctions SiH c'est-à-dire respectivement 20 % et 43 % en poids de fonction SiH par polymère.

Cependant, la problématique « tack » vis-à-vis de la peau n'est pas abordée par cette référence. En effet, dans le domaine médical ou paramédical, il est important que les gels silicones adhèrent bien à la peau car ils servent également de moyen pour fixer l'article à la peau de l'utilisateur et pour le maintenir en place dans de nombreux dispositifs médicaux. La force d'adhérence à la peau d'un gel silicone est évaluée par la mesure du «tack» ou collant instantané qui évalue l'habilité d'un gel silicone à adhérer rapidement à la peau.

Pour apprécier et évaluer le collant instantané ou « tack », une méthode dite de «Probe Tack» est connue et est décrite dans la norme ASTM D2979. Ce test permet de mesurer l'adhésion instantanée de l'adhésif. Le principe est le suivant pour les gels silicones décrits dans le présent mémoire: un poinçon cylindrique à face plane est amené en contact avec le film d'adhésif qui est déposé sur le substrat. Le poinçon est maintenu ensuite en contact avec le gel silicone pendant un temps de contact d'une seconde à une pression constante de 100 gf/cm². Ensuite, le poinçon est décollé à vitesse constante de 10 mm/s du gel, et la force nécessaire pour séparer le gel silicone de la tige est mesurée et s'exprime en gf/cm² alors que l'énergie de décollement elle s'exprime en mJ/cm². Dans le présent mémoire, lorsqu'il est fait référence à la propriété de « tack » à la peau, cette propriété est évaluée via l'énergie de décollement du gel testé. Ainsi, un gel silicone ayant un « tack » (ou énergie de décollement) supérieur ou égal à 14 mJ/cm mesuré selon les conditions décrites ci-dessus est un gel silicone particulièrement recherché et adapté pour une utilisation dans les dispositifs médicaux au contact de la peau.

Des dispositifs médicaux destinés à être en contact avec la peau et utilisant des gels silicones sont à présent très répandus. En effet, les propriétés intrinsèques des gels silicones font qu'ils adhèrent à une peau sèche, mais ne collent pas sur la surface d'une plaie humide, ne provoquant pas ainsi de dommage lors de leurs retraits. Cependant, il y a toujours un besoin pour améliorer le «tack» ou collant instantané des gels silicones ne serait-ce que pour maintenir le dispositif médical en place sur la peau du patient.

Les gels silicones présentent aussi l'avantage de pouvoir être assemblés à un grand nombre de supports tout en étant inertes pour l'organisme, évitant ainsi tout problème de toxicité lorsqu'ils sont utilisés chez l'être humain. Les gels silicones sont entre autres utilisés pour le traitement des plaies ou cicatrices car ils apportent au dispositif médical des propriétés facilitant la guérison du patient en maintenant un milieu humide autour de la plaie et permettent ainsi de maintenir l'hydratation des tissus lésés. Ces propriétés sont bien documentées et comprennent le fait que les gels silicones ne laissent pas de particules ou de fibres dans la plaie, sont souples sur la peau et sont confortables.

Ainsi, de nombreux dispositifs médicaux intègrent des gels silicones en tant qu'adhésif à la peau ou en tant que couche de contact d'une plaie à traiter et pour ces applications le substrat doit être biocompatible, flexible et avoir de bonnes propriétés mécaniques. Les films en polyuréthane sont très utilisés car ils répondent à l'ensemble de ces exigences techniques notamment :
- une bonne biocompatibilité,
- une bonne perméabilité à la vapeur d'eau (gestion des fluides entre le pansement et le milieu extérieur pour éviter au pansement de gonfler ou de se décoller),
- de bonnes propriétés mécaniques (résistance à la traction, capacité d'élongation),
- un toucher doux, et
- une bonne flexibilité.

La mise en œuvre de ces produits passe par une étape d'enduction sur un substrat. L'enduction des gels silicones sur ces supports en polyuréthane, est une étape clé du procédé de fabrication du dispositif médical et les propriétés de ce composite doivent être bonnes. En effet, le gel doit être suffisamment adhésif à la peau pour maintenir le dispositif en place et il doit aussi suffisamment adhérer au substrat pour éviter la génération de résidus au retrait du dispositif.

Cependant, il est connu que l'adhésion des gels silicones sur des supports en plastique ayant une faible énergie de surface est difficile à obtenir. Or l'adhésion au substrat est fondamentale pour assurer la bonne cohésion de l'article adhésif à la peau, notamment pour un usage médical ou paramédical, et éviter soit la présence de résidus au retrait de l'article, soit éviter la formation de poche d'air source de plis lorsque le gel est utilisé dans une prothèse mammaire comprenant une enveloppe en plastique.

Ainsi, lorsque des gels silicones sont utilisés soient comme éléments d'enduction de supports en plastique, par exemple en polyuréthane, ou soit dans une prothèse mammaire constituée d'une poche en plastique, par exemple en polyuréthane, il est souhaitable de renforcer leur adhérence aux supports de manière à ce que les produits les utilisant ne présentent plus les problèmes décrits ci-dessus.

Face à cette problématique, Il est connu que l'adhésion des gels silicone sur un substrat plastique est améliorée en appliquant sur la surface du support un traitement Corona afin de modifier l'énergie de surface dudit support. Cette technique consiste à oxyder la surface du matériau afin d'améliorer la mouillabilité en augmentant la tension de surface, Cependant, le niveau d'adhérence obtenu n'est pas toujours suffisant pour certaines applications.

Une autre voie pour améliorer l'adhérence des gels silicones sur des supports en plastique, par exemple en polyuréthane, consiste à utiliser un primaire d'adhérence, connus aussi sous le nom de « primaire d'accrochage ». Cette approche technique permet d'obtenir des produits offrant un niveau d'accroche légèrement amélioré par rapport à un substrat polymérique soumis à un traitement Corona, Parmi les primaires existants à la date on peut citer :
- Les primaires formulés en milieu solvant. Un exemple est décrit dans la demande de brevet WO 2011/092404 de la société Bluestar Silicones France où un primaire est constitué d'une matière active (organopolysiloxane à fonction hydrogénosilylé (SiH) et Si-alcényle ou une résine silicone ayant des fonctions hydrogénosilylé) diluée dans un solvant silicone (cyclopentasiloxane). Ce primaire est très performant mais doit être utilisé dans des conditions bien précises (dilution de matière active, poids de primaire enduit) pour qu'un bon compromis de propriétés (adhésion sur le substrat et conservation du « tack ») soit atteint. De plus, un autre inconvénient de ce type de primaire est sa teneur en solvant qui rend son utilisation plus difficile lors de l'étape d'enduction ; et
- les primaires en élastomère de silicone qui sont préparés à partir de compositions précurseurs réticulant par une réaction d'hydrosilylation comprenant des promoteurs d'adhésion qui sont le plus souvent des silanes qui permettent d'améliorer l'adhésion sur divers substrats (en polyamide, en polyester ou en polyuréthane). Cependant, lors de la préparation de l'élastomère silicone la condensation des silanes libère des produits secondaires (alcools) qui rendent l'utilisation de ce type de primaire plus difficile lors de l'étape d'enduction.

Il est également possible d'utiliser des primaires d'adhérence formulés en phase aqueuse mais les résultats en termes d'amélioration d'adhérence ne sont pas satisfaisants, et nécessite en outre l'incorporation d'additifs (comme par exemple de l'acide acétique) lors de leur mise en œuvre et/ou l'application d'un traitement thermique, traitement incompatible avec l'utilisation de certains substrats polymériques.

Selon une autre approche technique, par exemple décrite dans la demande de brevet WO-2005/051442 de la société Dow Corning Corp., l'adhérence d'un gel silicone à la surface d'un substrat polymérique plastique tels que des films en polyuréthane est améliorée en traitant directement le substrat ou le gel silicone par une mise en contact avec des dérivés: titanate, zirconate, siloxanes ayant des fonctions hydrogénosylilées ou du platine tel qu'un complexe platine-(0)-1,3-diéthenyl-1,1,3,3-tétraméthyldisiloxane. Il est à remarquer que l'utilisation de primaires d'adhérence de type titanate, tel que le tétrabutanoate de titane, est très répandue mais pose des problèmes lors de leur mise en œuvre.

Une solution au problème énoncé ci-dessus est exposé dans la demande de brevet WO 2014/131999 de la société Urgo qui décrit un article comprenant au moins un substrat polymérique assemblé à au moins une couche de polymère de silicone, caractérisé en ce que l'un au moins du substrat polymérique ou de la couche de polymère de silicone a été mis en contact avec des particules de dioxyde de titane, d'oxyde de magnésium et/ou d'oxyde de zinc, avant assemblage dudit article, et en ce que l'un au moins du substrat polymérique ou de la couche de polymère de silicone a été mis en contact avec de l'eau avant ou après assemblage dudit article. Les résultats d'adhésion présentent une amélioration comparée à un traitement Corona ou à l'imprégnation d'un support non tissé à base de polyéthylène imprégné avec du tétrabutanoate de titane à 5% en poids dans l'isopropanol.

Ainsi, l'utilisation de primaires d'adhérence de différents types ou de promoteurs d'adhésion de type titanate ou silanes dans les gels silicones engendrent bon nombre de problèmes ou de complications liés à leur mise en œuvre et à leur utilisation. En outre, les niveaux d'adhérence obtenus par ces traitements nécessitent encore d'être améliorés.

La Demanderesse a ainsi cherché à mettre au point un nouveau gel silicone offrant une adhésion améliorée entre un gel de silicone et un film en plastique, par exemple en polyuréthane ou en polyester, n'utilisant pas de promoteurs d'adhésion de type titanate ou silanes ni même de solvant et offrant les meilleures garanties possibles en matière de sécurité sanitaire.

Dans cet état de connaissance, l'un des objectifs essentiel de la présente invention est de fournir une composition silicone qui après réticulation fournie un gel silicone ayant :
- une bonne adhésion sur des matériaux en plastique, par exemple en polyester ou en polyuréthane, et
- une bonne adhésion (ou « tack ») sur la peau, c'est-à-dire un gel silicone ayant un collant instantanné (ou « tack ») supérieur ou égal à 14 mJ/cm mesuré selon les conditions décrites ci-dessus.

Un autre objectif de la présente invention est de proposer de nouveaux articles adhésifs à la peau comprenant un gel silicone selon l'invention présentant de bonnes propriétés de « tack » sur la peau.

Ces objectifs sont atteints par l'invention qui concerne une composition silicone A précurseur d'un gel silicone **G** et réticulable par hydrosilylation comprenant :
1) au moins un organopolysiloxane **B** comprenant:
   (i) au moins deux motifs siloxyles de formule (**B1**) :

      (Y)ₐ(Z)_{b}SiO(_{4-(a+b)/2} (**B1**)

      dans laquelle :
      - Y représente un radical monovalent contenant de 2 à 6 atomes de carbone, ayant au moins un groupe alcényle,
      - Z représente un radical monovalent contenant de 1 à 20 atomes de carbone et ne comprenant pas de groupe alcényle;
      - a et b représentent des nombres entiers, a valant 1, 2 ou 3, b valant 0, 1 ou 2 et (a+b) valant 1, 2 ou 3 ;
   (ii) et comportant éventuellement d'autres motifs siloxyles de formule (**B2**) :

      (Z)_{c}SiO_{(4-c)/2} (**B2**)

      dans laquelle :
      - Z a la même signification que ci-dessus, et
      - c représente un nombre entier valant 0, 1, 2 ou 3,
2) au moins un organopolysiloxane CE comprenant :
   - deux motifs siloxyles terminaux, identiques ou différents, de formule (**CE-1**):

      (H)ₚ(R¹)_{q}SiO_{1/2} (**CE-1**)

      dans laquelle ;
      - le symbole R¹ correspond à un groupe alkyle en C₁ à C₈ ou un groupe aryle en Ce à C₁₀ ;
      - et le symbole H représente un atome d'hydrogène, avec p= 0 ou 1, q= 2 ou 3 et (p+q)= 3;
   - au moins un motif siloxyle de formule (**CE-2**) ;

      (H)ₙ(R²)ₘSiO_{2/2} (**CE-2**)

      dans laquelle le radical R² correspond à un groupe alkyle en C₁ à C₈ ou un groupe aryle en C₆ à C₁₀, le symbole H représente un atome d'hydrogène, et n= 0 ou 1, m =1 ou 2 et (n+m)= 2, et
   - avec la condition selon laquelle l'organopolysiloxane **CE** contient deux atomes d'hydrogène liés chacun à un atome de silicium différent par polymère, et de préférence l'organopolysiloxane **CE** contient par polymère deux motifs siloxyles de formule (**CE-1**) dans laquelle p=1 et au moins un motif siloxyle de formule (CE-2) dans laquelle n=0 ;
3) au moins un organopolysiloxane **XL** comprenant :
   - au moins trois motifs siloxyles de formule (**XL-1**);

      (H) (L)ₑ SiO_{(3-e)/2} (**XL-1**)

      dans laquelle le symbole H représente un atome d'hydrogène, le symbole L représente un alkyle ayant de 1 à 8 atomes de carbone inclus ou un aryle en C₆ à C₁₀, et le symbole e est égal à 0, 1 ou 2 ; et
   - éventuellement des autres motifs siloxyles de formule (**XL-2**):

      (L)_{g} SiO_{(4-g)/2} (**XL-2**)

      dans laquelle le symbole L représente un alkyle ayant de 1 à 8 atomes de carbone inclus ou un aryle en C₆ à C₁₀ et le symbole g est égal à 0,1, 2 ou 3, et
   - avec la condition selon laquelle l'organopolysiloxane **XL** contient entre 15,1 % et 40,0 % en poids de fonction Si-H par polymère, et de préférence entre 18 % et 35,0 % en poids de fonction Si-H par polymère,
4) une quantité efficace d'au moins un catalyseur d'hydrosilylation **E,**
5) au moins un inhibiteur **D** de la réaction d'hydrosilylation,
6) éventuellement au moins un additif **K,** et
   les quantités en poids des organopolysiloxanes **B, CE** et **XL** sont choisies de manière à satisfaire les trois conditions suivantes :
   a) le rapport molaire RHAlk = tH/tAlk à 3,0 de préférence 3,0 s RHAlk S24 et encore plus préférentiellement entre 4,5 ≤ RHAlk ≤ 20;
   b) le rapport molaire RH^{CE}V = nH^{CE}/tAlk ≥ 4,50, et de préférence 4,50 ≤ RH^{CE}V ≤ 20, et
   c) le % molaire RH^{CE} = (nH^{CE}/tH) x 100 est supérieur ou égal à 90 % molaire, et de préférence compris entre 90% molaire et 95% molaire, avec :
      - tH = nombre de moles d'atome d'hydrogène directement lié à un atome de silicium des organopolysiloxanes **CE** et **XL,**
      - tAlk = nombre de mole d'alcényle directement lié à un atome de silicium de l'organopolysiloxane **B** ; et
      - nH^{CE} = nombre de mole d'atome d'hydrogène directement lié à un atome de silicium de l'organopolysiloxane **CE,**

La Demanderesse a mis en œuvre d'importants moyens de recherche et de nombreuses expérimentations pour atteindre cet objectif parmi d'autres. Et au terme de cela, elle a eu le mérite de trouver, de manière tout à fait surprenante et inattendue, que pour obtenir un gel silicone ayant une bonne adhésion à un support en plastique (par exemple en polyuréthane) et un bon « tack » à la peau (supérieur ou égal à 14 mJ/cm mesuré selon les conditions décrites ci-dessus), il suffit de mettre en œuvre des compositions silicones réticulant par des réactions d'hydrosilylation comprenant des organopolysiloxanes XL ayant un taux en fonction SiH spécifique, compris entre 15,1 % et 40,0 % en poids de fonction Si-H par polymère, de préférence compris entre 18 % et 35,0 % en poids de fonction Si-H par polymère, et que le choix des constituants soit déterminé de manière à respecter les conditions suivantes
a) le rapport molaire RHAlk = tH/tAlk ≥ 3,0,
b) le rapport molaire RH^{CE}V = nH^{CE}/tAlk ≥ 4,50, et
c) le % molaire RH^{CE} = (nH^{CE}/tH) x 100 est supérieur ou égal à 90 % molaire, et de préférence compris entre 90% molaire et 95% molaire.

Au sens de la présente invention, l'expression « gel silicone » désigne un produit silicone réticulé ne présentant aucun écoulement lorsqu'il est à l'état stable et est caractérisé notamment par un taux de pénétration (ou « pénétrabilité ») compris entre 80 et 300 dixièmes de mm. Elle se mesure par pénétrométrie selon la norme NF ISO 2137, à l'aide d'un pénétromètre modèle PNR 12 Petrotest avec un poids total de la tige et du cône fixé à 62,5 g. La pénétrabilité au cône d'un gel silicone est déterminée à 25 °C en mesurant la profondeur de pénétration du cône dans l'échantillon, celle-ci étant obtenue en libérant l'ensemble cône du pénétromètre et en laissant le cône agir pendant 5 secondes.

Selon un mode de réalisation particulier de la composition silicone **A:**
- l'organopolysiloxane **B** à une viscosité dynamique à 25°C comprise entre 100 mPa.s et 120000 mPa.s,
- l'organopolysiloxane **CE** à une viscosité dynamique à 25°C comprise entre 1 mPa.s et 500 mPa.s, et de préférence entre 5 et 200 mPa.s, et
- l'organopolysiloxane **XL** à une viscosité dynamique à 25°C comprise entre 5 mPa.s et 2000 mPa.s, et de préférence entre 5 et 500 mPa.s.

Toutes les viscosités dont il est question dans le présent exposé correspondent à une grandeur de viscosité dynamique à 25°C dite "Newtonienne", c'est-à-dire la viscosité dynamique qui est mesurée, de manière connue en soi, avec un viscosimètre Brookfield à un gradient de vitesse de cisaillement suffisamment faible pour que la viscosité mesurée soit indépendante du gradient de vitesse.

Selon un mode de réalisation avantageux, la nature et les quantités en poids les quantités en poids des organopolysiloxanes **B, CE** et **XL** sont choisis de manière à ce que la viscosité dynamique à 25°C de la composition silicone **A** soit comprise entre 200 mPa.s et 100000 mPa.s, et de préférence comprise entre 200 mPa.s et 80000 mPa.s.

Selon l'invention, il est avantageux de respecter les deux conditions suivantes :
- l'organopolysiloxane **CE** a au moins 5 atomes de silicium et un ratio; (nombre de mole de groupe SiH)/(nombre total d'atome de silicium) compris entre 0,05 et 0,40, et de préférence compris entre 0,08 et 0,35, et
- l'organopolysiloxane **XL** a au moins 5 atomes de silicium et un ratio: (nombre de mole de groupe SiH)/(nombre total d'atome de silicium) compris entre 0,35 et 0,80, et de préférence compris entre 0,35 et 0,75.

Selon un autre mode de réalisation particulier, la composition silicone **A** selon l'invention comprend au moins deux organopolysiloxanes **B** comportant, par molécule, au moins deux radicaux alcényles en C₂ à Ce liés chacun à un atome de silicium, le premier ayant une viscosité dynamique à 25°C comprise entre 50000 mPa.s et 120000 mPa.s, et le deuxième ayant une viscosité dynamique à 25°C comprise entre 500 mPa.s et 20000 mPa.s.

Selon l'invention, il est judicieux que pour la définition de l'organopolysiloxane **B** dans la formule (**B1**), le symbole a peut préférentiellement valoir 1 ou 2, et encore plus préférentiellement 1. De plus, dans la formule (**B1**) et dans la formule (**B2**), le symbole **Z** peut représenter préférentiellement un radical monovalent choisi dans le groupe constitué par un groupe alkyle ayant 1 à 8 atomes de carbone, éventuellement substitué par au moins un atome d'halogène, et un groupe aryle en C₆ à C₁₀. **Z** peut avantageusement représenter un radical monovalent choisi dans le groupe constitué par ; méthyle, éthyle, propyle, 3,3,3-trifluoropropyle, xylyle, tolyle et phényle. En outre, dans la formule (B1), le symbole Y peut avantageusement représenter un radical choisi dans le groupe constitué par vinyle, propényle, 3-butényle et 5-hexényle. De préférence, le symbole Y est un vinyle et le symbole **Z** est un méthyle.

L'organopolysiloxane **B** peut présenter une structure linéaire, ramifié, cycliques ou en réseau. Lorsqu'il s'agit d'organopolysiloxanes linéaires, ceux-ci peuvent être essentiellement constitués ;
- de motifs siloxyles « D » choisi parmi les motifs de formules (Y)₂SiO_{2/2}, (Y)(Z)SiO_{2/2} et (Z)₂SiO_{2/2}; et
- de motifs siloxyles « M » choisis parmi les motifs de formules (Y)₃SiO_{1/2}, (Y)₂(Z)SiO_{1/2}, (Y)(Z)₂SiO_{1/2} et (Z)₃SiO_{2/2},
- formules dans lesquelles les symboles **Y** et **Z** sont tels définis ci-dessus.

A titre d'exemples de motifs « **D** », on peut citer les groupes diméthylsiloxy, méthylphénylsiloxy, méthylvinylsiloxy, méthylbuténylsiloxy, méthylhexénylsiloxy, méthyldécénylsiloxy et méthyldécadiénylsiloxy.

A titre d'exemple de motifs « M », on peut citer les groupes triméthylsiloxy, diméthylphénylsiloxy, diméthylvinylsiloxy et diméthylhexénylsiloxy.

L'organopolysiloxane **B,** en particulier lorsqu'il est linéaire, peut-être une huile silicone ayant une viscosité dynamique à 25°C comprise entre 50 mPa.s et 120 000 mPa.s, et préférentiellement entre 100 mPa.s et 120 000 mPa.s.

Lorsque l'organopolysiloxane **B** est cyclique, celui-ci peut être constitué de motifs siloxyles « D » choisi parmi les motifs de formules Y₂SiO_{2/2}, YZSiO_{2/2} et Z₂SiO_{2/2}. Des exemples de tels motifs « D » sont décrits ci-dessus. Cet organopolysiloxane cyclique peut avoir une viscosité dynamique à 25°C comprise entre 1 mPa.s et 5 000 mPa.s.

Comme exemples d'organopolysiloxane **B** utiles on peut citer :
- les polydiméthylsiloxanes à extrémités diméthylvinylsilyles ;
- les poly(méthylphénylsiloxane-co-diméthylsiloxane) à extrémités diméthylvinylsilyles ;
- les poly(vinylméthylsiloxane-co-diméthylsiloxane) à extrémités diméthylvinylsilyles ;
- les po|y(diméthylsiloxane-co-vinylméthylsiloxane) à extrémités triméthylsilyles ;
- et les polyméthylvinylsiloxanes cycliques.

Les organopolysiloxanes **B** qui sont des polydiméthylsiloxanes à extrémités diméthylvinylsilyles ayant une viscosité dynamique à 25°C comprise entre 50 mPa.s et 120 000 mPa.s, et de préférence comprise entre 100 mPa.s et 80000 mPa.s sont particulièrement avantageux. Les organopolysiloxanes **B** particulièrement avantageux sont ceux de formule M^{VI}ₓM^{VI} dans laquelle :
- M^{VI} = motif siloxyle de formule : (vinyle)(CH₃)₂SiO_{1/2}
- D = motif siloxyle de formule : (CH₃)₂SiO_{2/2}. et
- x est un nombre compris entre 1 et 1000, et de préférence compris entre 5 et 1000.

Comme exemples d'organopolysiloxane **CE** qui a une fonction «d'allongeur de chaîne » on peut citer les polydiméthylsiloxanes à extrémités diméthylhydrogénosilyles ayant une viscosité dynamique à 25°C comprise entre 1 mPa.s et 500 mPa.s, de préférence comprise entre 5 mPa.s et 200 mPa.s, et encore plus préférentiellement comprise entre 1 et 30 mPa.s. Des organopolysiloxanes **CE** particulièrement avantageux sont les poly(diméthylsiloxy)α,ω(dirnéthylhydrogénosiloxy de formule M^{H}DₓM^{H} dans laquelle ;
- M^{H} = motif siloxyle de formule : (H)(CH₃)₂SiO_{1/2}
- D = motif siloxyle de formule : (CH₃₎₂SiO_{2/2}, et
- x est un nombre compris entre 1 et 200, et de préférence compris entre 1 et 150, et encore plus préférentiellement compris entre 3 et 120.

L'organopolysiloxane **CE** est qualifié « d'allongeur de chaine » car il a pour effet présumé d'augmenter la taille des mailles du réseau au cours de la réticulation. Lorsque les fonctions réactives SiH sont en bout de chaîne, le terme « téléchélique » est parfois préféré au terme « allongeur de chaine »,

Comme organopolysiloxane XL qui a une fonction de réticulant et qui est utile selon l'invention on peut citer ceux de formules M^{H} Dₓ D_{W}^{H} M^{H}, M^{H} Dₓ D_{y}^{H} M et M Dₓ D_{z}^{H} M, formules dans lesquelles :
- M^{H} = motif siloxyle de formule : (H)(CH₃)₂SiO_{1/2}
- D^{H} = motif siloxyle de formule : (H)(CH₃) SiO_{2/2}
- D = motif siloxyle de formule : (CH₃)₂SiO_{2/2}; et
- M = motif siloxyle de formule : (CH₃)₃SiO_{1/2}
- avec :
   - x est un nombre compris entre 0 et 500, de préférence entre 2 et 250, et encore plus préférentiellement entre 5 et 40,
   - w est un nombre compris entre 1 et 500, de préférence compris entre 1 et 250 ou entre 1 et 100, et encore plus préférentiellement compris entre 1 et 70 ;
   - y est un nombre compris entre 2 et 500, de préférence compris entre 3 et 250 ou entre 2 et 100, et encore plus préférentiellement compris entre 2 et 70 ; et
   - z est un nombre compris entre 3 et 500, de préférence compris entre 3 et 250 ou entre 3 et 100, et encore plus préférentiellement compris entre 3 et 70, et
   - avec la condition que l'organopolysiloxane **XL** contient entre 15,1 % et 40,0 % en poids de fonction Si-H par polymère, et de préférence entre 18 % et 35,0 % en poids de fonction Si-H par polymère.

Comme catalyseur d'hydrosilylation **E** utile selon l'invention, on peut citer les composés d'un métal appartenant au groupe du platine bien connu de l'homme de l'art, Les métaux du groupe du platine sont ceux connus sous le nom de platinoïdes, appellation qui regroupe, outre le platine, le ruthénium, le rhodium, le palladium, l'osmium et l'iridium. On utilise, de préférence, les composés du platine et du rhodium. On peut, en particulier, utiliser les complexes du platine et d'un produit organique décrit dans les brevets US-A-3 159 601, US-A-3 159 602, US-A-3 220 972 et les brevets européens EP-A-0 057 459, EP-A-0 188 978 et EP-A-0 190 530, les complexes du platine et d'organosiloxanes vinylés décrits dans les brevets US-A-3 419 593. Le catalyseur généralement préféré est le platine. A titre d'exemples, on peut citer le platine noir, l'acide chloroplatinique, un acide chloroplatinique modifié par un alcool, un complexe de l'acide chloroplatinique avec une oléfine, un aldéhyde, un vinylsiloxane ou un alcool acétylénique, entre autres. La préférence va à la solution ou complexe de Karstedt, tel que décrit dans le brevet US-A-3 775 452, à l'acide chloroplatinique hexahydrate ou un catalyseur au platine comprenant des ligands carbènes.

Comme inhibiteur **D** de la réaction d'hydrosilylation utile selon l'invention, on peut citer celui choisi parmi les alcools α-acétyléniques, les diesters α-α'-acétyléniques, les composés conjugués ène-yne, les cétones α-acétyléniques, les acrylonitriles, les maléates, les fumarates et les mélanges de ceux-ci. Ces composés capables de remplir la fonction d'inhibiteur d'hydrosilylation sont bien connus de l'homme du métier. Ils peuvent être utilisés seuls ou en mélanges.

Un inhibiteur **D** de type alcool α-acétylénique peut être choisi parmi les composés de formule (D1) suivante :

(R¹)(R²)C(OH)-C≡CH (D1)

dans laquelle :
- le groupe R¹ représente un groupe alkyle, un groupe cycloalkyle, un groupe (cycloalkyl)alkyle, un groupe aryle en C₆ C₁₀ ou un groupe arylalkyle en C₇ à C₁₈,
- le groupe R² représente un atome d'hydrogène, un groupe alkyle, un groupe cycloalkyle, un groupe (cycloalkyl)alkyle, un groupe aryle en C₆ à C₁₀ ou un groupe arylalkyle en C₇ à C₁₈,
- ou bien R¹ et R² constituent ensemble avec l'atome de carbone auquel ils sont liés un cycle aliphatique à 5, 6, 7 ou 8 chaînons, éventuellement substitué une ou plusieurs fois.

Selon la formule (D1) :
- par « alkyle », on entend une chaîne hydrocarbonée saturée contenant de 1 à 20 atomes de carbone, de préférence de 1 à 8 atomes de carbone. Un groupe alkyle peut être choisi dans le groupe constitué par méthyle, éthyle, isopropyle, n-propyle, tert-butyle, isobutyle, n-butyle, n-pentyle, isoamyle et 1,1 -diméthylpropyle ;
- par « cycloalkyle », on entend selon l'invention un groupe hydrocarboné saturé monocyclique ou polycyclique, de préférence monocyclique ou bi cyclique, contenant de 3 à 20 atomes de carbone, de préférence de 5 à 8 atomes de carbone. Lorsque le groupe cycloalkyle est polycyclique, les multiples noyaux cycliques peuvent être rattachés les uns aux autres par une liaison covalente et/ou par un atome spinanique et/ou être condensés les uns aux autres. Un groupe cycloalkyle peut être choisi dans le groupe constitué par le cyclopropyle, le cyclobutyle, le cyclopentyle, le cyclohexyle, le cycloheptyle, le cyclooctyle, l'adamantane et le norborane ;
- par « (cycloalkyl)alkyle », on entend selon l'invention un groupe cycloalkyle tel que défini ci-avant lié à un groupe alkyle tel que défini ci-avant également ;
- Par « aryle », on entend selon l'invention un groupe hydrocarboné aromatique contenant de 6 à 10 atomes de carbone, monocyclique ou polycyclique. Un groupe aryle peut être choisi dans le groupe constitué par phényle, naphtyle et anthracényle;
- par « arylalkyle », on entend selon l'invention un groupe aryle tel que défini ci-avant lié à un groupe alkyle tel que défini ci-avant également.

Selon un mode de réalisation préféré, dans la formule (D1) R¹ et R² constituent ensemble avec l'atome de carbone auquel ils sont liés un cycle aliphatique non substitué à 5, 6, 7 ou 8 chaînons. Selon un autre mode de réalisation préféré, R¹ et R², identiques ou différents, représentent indépendamment l'un de l'autre un groupe alkyle monovalent en C₁ à C₁₂, de préférence en C₁ à C₆.

Un inhibiteur **D** qui est un alcool α-acétylénique utile selon l'invention peut être choisi dans le groupe constitué par les composés suivants : 1-éthynyl-1-cyclopentanol ; 1-éthynyl-1-cyclohexanol (aussi appelé ECH) ; 1-éthynyl-1-cycloheptanol ; 1-éthynyl-1-cyclooctanol ; 3-méthyl-1-butyn-3-ol (aussi appelé MBT) ; 3-méthyl-1-pentyn-3-ol ; 3-méthyl-1-hexyn-3-ol ; 3-méthyl-1-heptyn-3-ol ; 3-méthyl-1-octyn-3-ol ; 3-méthyl-1-nonyn-3-ol ; 3-méthyl-1-decyn-3-oi ; 3-méthyl-1-dodecyn-3-ol; 3-méthyl-1-pentadecyn-3-ol ; 3-éthyl-1-pentyn-3-ol ; 3-éthyl-hexyn-3-ol ; 3-éthyl-1-heptyn-3-ol ; 3,5-diméthyl-1-hexyn-3-ol ; 3-isobutyl-5-méthyl-1-hexyn-3-ol ; 3,4,4-triméthyl-1-pentyn-3-ol ; 3-éthyl-5-méthyl-1-heptyn-3-ol ; 3,6-diéthyl-1-nonyn-3-ol; 3,7,11-triméthyl-1-dodecyn-3-ol (aussi appelé TMDDO); 1,1-diphényl-2-propyn-1-ol ; 3-butyn-2-ol ; 1-pentyn-3-ol ; 1-hexyn-3-ol ; 1-heptyn-3-ol ; 5-méthyl-1-hexyn-3-ol ; 4-éthyl-1-octyn-3-ol et 9-éthynyt-9-fluorenol.

Un inhibiteur **D** de type diester α-α'-acétylénique peut être choisi parmi les composés de formule (D2) suivante : 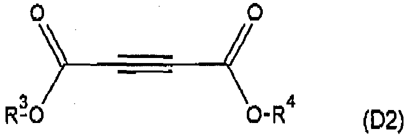 dans laquelle les groupe R³ et R⁴, identiques ou différents, représentent indépendamment l'un de l'autre un groupe alkyle, un groupe cycloalkyle, un groupe (cycloalkyl)alkyle, un groupe aryle en C₆ à C₁₀, un groupe arylalkyle en C₇ à C₁₈ ou un groupe silyle.

Par « silyle », on entend selon l'invention un groupe de formule -SiR₃, dans laquel chaque symbole R représente indépendamment un groupe alkyle contenant de 1 à 20 atomes de carbone, de préférence de 1 à 8 atomes de carbone. Un groupe silyle peut être par exemple le groupe triméthylsilyle.

Selon un mode de réalisation particulier, dans la formule (D2) R³ et R⁴, identiques ou différents, représentent indépendamment l'un de l'autre un groupe alkyle en C₁ à C₁₂, de préférence en C₁ à C₆, ou le groupe triméthylsilyle. Un inhibiteur **D** qui est un diester α-α'-acétylénique utile selon l'invention peut être choisi dans le groupe constitué par les composés suivants: l'acétylène-dîcarboxylate de diméthyle (DMAD), l'acétylène-dicarboxylate de diéthyle, l'acétylène-dicarboxyiate de tert-butyle et l'acétylène-dicarboxylate de bis(triméthylsilyle).

Un inhibiteur **D** de type composé conjugué ène-yne peut être choisi parmi les composés de formule (D3) suivante ; 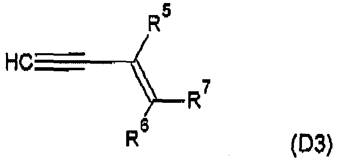 dans laquelle ;
- les groupes R⁵, R⁶ et R⁷ représentent, indépendamment les uns des autres, un atome d'hydrogène, un groupe alkyle, un groupe cycloalkyle, un groupe (cycloalkyl)alkyle, un groupe aryle en C₆ à C₁₀ ou un groupe arylalkyle en C₇ à C₁₈,
- ou bien au moins deux groupes parmi les groupes R⁵, R⁶ et R⁷ constituent ensemble avec le ou les atomes de carbone auxquels ils sont liés un cycle aliphatique à 5, 6, 7 ou 8 chaînons, éventuellement substitué une ou plusieurs fois.

Selon un mode de réalisation particulier, les groupes R⁵, R⁶ et R⁷ représentent, indépendamment les uns des autres, un atome d'hydrogène, un groupe alkyle en C₁ à C₁₂, de préférence en C₁ à C₆, ou un groupe aryle en C₆ à C₁₀. Un inhibiteur **D** qui est un composé conjugué ène-yne utile selon l'invention peut être choisi dans le groupe constitué par les composés suivants : le 3-méthyl-3-pentène-1-yne ; le 3-méthyl-3-hexène-1-yne ; le 2,5-diméthyl-3-hexène-1-yne ; le 3-éthyl-3-butène-1-yne ; et le 3-phényl-3-butène-1-yne. Selon un autre mode de réalisation particulier, deux groupes choisis parmi les groupes R⁵, R⁶ et R⁷ constituent ensemble avec le ou les atomes de carbone auxquels ils sont liés un cycle aliphatique non substitué à 5, 6, 7 ou 8 chaînons et le troisième groupe restant représente un atome d'hydrogène ou un groupe alkyle en C₁ à C₁₂, de préférence en C₁ à C₆. Un inhibiteur D qui est un composé conjugué ène-yne utile selon l'invention peut être le 1-éthynyl-1-cyclohexène,

Un inhibiteur **D** de type cétone α-acétylénique peut être choisi parmi les composés de formule (D4) suivante : 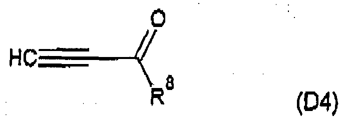 dans laquelle : R⁸ représente un groupe alkyle, un groupe cycloalkyle, un groupe (cycloalkyl)alkyle, un groupe aryle en C₆ à C₁₀ ou un groupe arylalkyle en C₇ à C₁₈, les groupes alkyles, cycloalkyles, (cycloalkyl)alkyle, aryle ou arylalkyle pouvant éventuellement être substitué une ou plusieurs fois par un atome de chlore, de brome ou d'iode.

Selon un mode de réalisation préféré, R⁸ représente un groupe alkyle monovalent en C₁ à C₁₂, de préférence en C₁ à C₆, éventuellement être substitué une ou plusieurs fois par un atome de chlore ou de brome, ou un groupe cycloalkyle, ou un groupe aryle en C₆ à C₁₀. Un inhibiteur D qui est une cétone α-acétylénique utile selon l'invention peut être choisi dans le groupe constitué par les composés suivants : la 1-octyn-3-one, la 8-chloro-1-octyn-3-one ; la 8-bromo-1-octyn-3-one ; la 4,4-diméthyl-1-octyn-3-one ; la 7-chloro-1-heptyn-3-one ; la 1-hexyn-3-one ; la 1-pentyn-3-0ne ; la 4-méthyl-1-pentyn»3-one ; la 4,4-diméthyl-1-pentyn-3-one ; la 1-cyclohexyl-1-propyn-3-one ; le benzoacétylène et le o-chlorobenzoyl-acétylène,

Un inhibiteur D de type acrylonitrile peut être choisi parmi les composés de formule (D5) suivante : 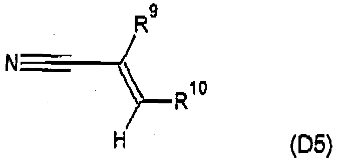 dans laquelle : R⁹ et R¹⁰ représentent, indépendamment l'un de l'autre, un atome d'hydrogène, un atome de chlore, de brome ou d'iode, un groupe alkyle, un groupe cycloalkyle, un groupe (cycloalkyl)alkyle, un groupe aryle en C₆ à C₁₀ ou un groupe arylalkyle en C₇ à C₁₈, les groupes alkyles, cycloalkyles, (cycloalkyl)alkyle, aryle ou arylalkyle pouvant éventuellement être substitué une ou plusieurs fois par un atome de chlore, de brome ou d'iode.

Un inhibiteur D qui est un acrylonitrile utile selon l'invention peut être choisi dans le groupe constitué par les composés suivants : l'acrylonitrile ; le méthacrylonitrile ; le 2-chloroacrylonitryle ; le crotononitrile et le cinnamonitrile.

Un inhibiteur D de type maléate ou fumarate peut être choisi parmi les composés de formules (D6) et (D7) suivantes : 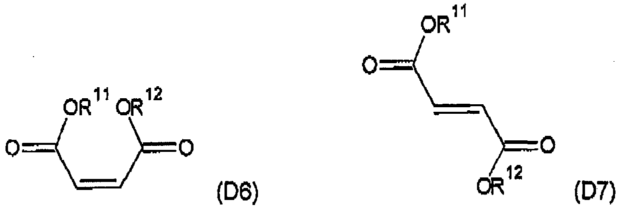 dans lesquelles ; R¹¹ et R¹², identiques ou différents, représentent indépendamment l'un de l'autre un groupe alkyle ou alcényle, un groupe cycloalkyle, un groupe (cycloalkyl)alkyle, un groupe aryle en C₆ à C₁₀ ou un groupe arylalkyle en C₇ à C₁₈, lesdits groupes alkyles, alcényles, cycloalkyles, (cycloalkyl)alkyles, aryles et arylalkyles pouvant être substitués par un groupe alcoxy

Par le terme « alcényle », on entend selon l'invention une chaîne hydrocarbonée saturée contenant de de 2 à 6 atomes de carbone et comprenant au moins une double insaturation. De préférence, le groupe alcényle est choisi dans le groupe constitué par un vinyle ou un allyle.

Par « alcoxy », on entend selon les formules (D6) ou (D7), un groupe alkyle tel que défini ci-avant lié à un atome d'oxygène. Un groupe alcoxy peut être choisi dans le groupe constitué par méthoxy, éthoxy, propoxy et butoxy.

Selon un mode de réalisation particulier, R₁₁ et R₁₂, identiques ou différents, représentent indépendamment l'un de l'autre un groupe alkyle ou alcényle en C₁ à C₁₂, de préférence en C₁ à C₆, éventuellement substitué par un groupe alcoxy en C₁ à C₆.

Un inhibiteur **D** qui est un maléate ou un fumarate utile selon l'invention peut être choisi dans le groupe constitué par le fumarate de diéthyle, le maléate de diéthyle, le fumarate de diallyle, le maléate de diallyle et le maléate de bis-(méthoxyisopropyle).

Des inhibiteurs **D** choisis parmi les alcools α-acétyléniques, les diesters α-α'-acétyléniques, les composés conjugués ène-yne, les cétones α-acétyléniques, les acrylonitriles, les maléates, les fumarates sont disponibles dans le commerce. On peut citer notamment l'ECH (1-éthynyl-1 -cyclohexanol) qui est disponible commercialement chez BASF, le maléate de diméthyle qui est disponible commercialement chez DMS et l'acétylène-dicarboxylate de diméthyle qui est disponible chez City Chemical LLC.

Ces inhibiteurs sont ajoutés en quantité en poids comprise entre 1 et 50 000 ppm par rapport au poids de la composition silicone totale, notamment entre 10 et 10 000 ppm, de préférence entre 20 et 2000 ppm et encore plus préférentiellement entre 20 ppm et 500 ppm.

Comme exemple d'additif K on peut par exemple citer un stabilisant issue de la famille des dérivés silylés de l'acide phosphorique tels que les esters silylés de l'acide phosphorique.

Des résultats particulièrement avantageux sont obtenus lorsque la composition silicone A comprend:
1) au moins un organopolysiloxane **B** qui est un polydiméthylsiloxane à extrémités diméthylvinylsilyles ayant une viscosité dynamique à 25°C comprise entre 50 mPa.s et 120000 mPa.s, et de préférence comprise entre 100 mPa.s et 80000 mPa.s et ayant une formule M^{VI}DₓM^{VI} avec;
   - M^{VI} = motif siloxyle de formule : (vinyle)(CH₃)₂SiO_{1/2}.
   - D = motif siloxyle de formule : (CH₃)₂SiO_{2/2}, et
   - x est un nombre compris entre 0 et 1000, et de préférence compris entre 5 et 1000,
2) au moins un organopolysiloxane **CE** de formule M^{H}DₓM^{H} avec :
   - M^{H} = motif siloxyle de formule : (H)(CH₃)₂SiO_{1/2}
   - D = motif siloxyle de formule : (CH₃)₂SiO_{2/2}, et
   - x est un nombre compris entre 1 et 100, et de préférence compris entre 1 et 50, et encore plus préférentiellement compris entre 3 et 30,
3) au moins un organopolysiloxane **XL** de formule M^{H} Dₓ D_{w}^{H} M^{H}, M^{H} Dₓ D_{y}^{H} M ou M Dₓ D_{z}^{H} M, formules dans lesquelles :
   - M^{H} = motif siloxyle de formule : (H)(CH₃)₂SiO_{1/2}
   - D^{H} = motif siloxyle de formule : (H)(CH₃) SiO_{2/2}
   - D = motif siloxyle de formule : (CH₃)₂SiO_{2/2}: et
   - M = motif siloxyle de formule : (CH₃)₃SiO_{1/2}
   avec:
   - x est un nombre compris entre 0 et 500, de préférence entre 2 et 250, et encore plus préférentiellement entre 5 et 80,
   - w est un nombre compris entre 1 et 500, de préférence compris entre 1 et 250 ou entre 1 et 100, et encore plus préférentiellement compris entre 1 et 70 ;
   - y est un nombre compris entre 2 et 500, de préférence compris entre 3 et 250 ou entre 2 et 100, et encore plus préférentiellement compris entre 2 et 70 ; et
   - z est un nombre compris entre 3 et 500, de préférence compris entre 3 et 250 ou entre 3 et 100, et encore plus préférentiellement compris entre 3 et 70.
4) au moins un catalyseur **E** d'hydrosilylation,
5) au moins un inhibiteur **D** de la réaction d'hydrosilylation,
6) éventuellement au moins un additif **K,** et
   - avec la condition que l'organopolysiloxane **XL** contient entre 15,1 % et 40,0 % en poids de fonction Si-H par polymère, et de préférence entre 18 % et 35,0 % en poids de fonction Si-H par polymère, et
      les quantités en poids des organopolysiloxanes **B, CE** et **XL** sont choisies de manière à satisfaire les trois conditions suivantes :
      a) le rapport molaire RHAlk = tH/tAlk ≥ 3,0 de préférence 3,0 ≤ RHAlk ≤24 et encore plus préférentiellement entre 4,5 ≤ RHAlk ≤ 20;
      b) le rapport molaire RH^{CE}V = nH^{CE}/tAlk ≥ 4,50, et de préférence 4,50 ≤ RH^{CE}V ≤ 20, et
      c) le % molaire RH^{CE} = (nH^{CE}/tH) x 100 est supérieur ou égal à 90 %, et de préférence compris entre 90% et 95%, avec :
         - tH = nombre de moles d'atome d'hydrogène directement lié à un atome de silicium des organopolysiloxanes **CE** et **XL,**
         - tAlk = nombre de mole d'alcényle directement lié à un atome de silicium de l'organopolysiloxane **B ;** et
         - nH^{CE} = nombre de mole d'atome d'hydrogène directement lié à un atome de silicium de l'organopolysiloxane **CE.**

Un autre objet de l'invention concerne un gel silicone **G** obtenu par réticulation de la composition **A** selon l'invention et telle que définie ci-dessus, de préférence par chauffage à une température comprise entre 70°C et 200°C et/ou sous l'action d'un rayonnement infrarouge.

Le gel silicone **G** présente l'avantage d'adhérer parfaitement à un support par exemple en polyuréthane et de présenter un bon « tack » à la peau (ou) c'est-à-dire qu'il présente une valeur d'énergie de décollement ≥ 14 mJ/cm².

Un autre objet de l'invention concerne l'utilisation du gel silicone G selon l'invention et tel que défini ci-dessus dans les dispositifs médicaux tels qu'une prothèse mammaire, un pansement adhésif à la peau ou un dispositif de maintien d'accessoires médicaux utilisés au contact de la peau de type capteur, sonde, cathéter ou aiguille.

Un autre objet de l'invention concerne un article adhésif sur la peau comprenant un substrat **S** qui est un film en plastique, de préférence un film en polyester ou en polyuréthane, enduit de manière continue ou discontinue sur au moins une des deux faces par un gel silicone **G** selon l'invention et tel que défini ci-dessus.

Ainsi dans un mode de réalisation préféré, la composition silicone **A** est enduite de manière continue sur au moins une des deux faces dudit substrat S à raison d'une quantité comprise entre 20 et 500 g/m², de préférence comprise entre 40 et 350 g/m² et encore plus préférentiellement comprise entre 80 et 250 g/m² avant d'être réticulée, de préférence par chauffage à une température comprise entre 70°C et 200°C et/ou sous l'action d'un rayonnement infrarouge, de manière à obtenir ledit article adhésif sur la peau.

De préférence le substrat **S** est un film souple perforé en polyuréthane ou un film souple continu en polyuréthane. Ce film souple en polyuréthane peut-être fabriqué à partir de polyuréthane fondu soufflé.

Préférentiellement, on utilise un film souple en polyuréthane transparent ou translucide. Lorsque l'article adhésif a un usage de pansement, l'utilisation d'un film transparent ou translucide présente l'avantage de permettre l'observation de la plaie, de la blessure ou du site d'entrée d'un cathéter sur lequel le pansement doit être centré.

De préférence, le dit substrat S est un film souple en polyuréthane ayant une épaisseur de 5 à 600 µm, de préférence de 5 à 250 µm et encore plus préférentiellement de 10 à 100 µm. Comme exemple de film souple en polyuréthane on peut citer ceux qui sont utilisés dans les pansements commercialisés par la société Smith & Nephew sous la marque Opsite®, ou par la société 3M sous la marque Tegaderm® ou encore par les Laboratoires URGO sous la marque Optiskin®. Ces pansements sont constitués d'un film mince (de l'ordre de 20 à 50 µm) transparent de polyuréthane adhésivé. Leur transparence permet un contrôle visuel de la zone à traiter. Comme autre exemple de film souple en polyuréthane on peut aussi citer ceux commercialisés sous les marques Platilon® vendus par la société Bayer Material Science et Inspire® vendus par la société Coveris Advanced Coatings.

Selon un mode de réalisation préféré ledit substrat S est un film souple en polyuréthane continu qui est perméable à l'air et imperméable aux fluides. Ce film souple en polyuréthane peut présenter un taux de transmission de vapeur d'eau (MVTR : « Moisture Vapor Transmission Rate ») variable suivant l'application visée. Une technique de mesure du taux de transmission de vapeur d'eau en contact liquide est décrite dans la norme NF-EN 13726-2. De préférence, le film souple en polyuréthane sera choisi de manière à obtenir un pansement ayant un taux de transmission de vapeur d'eau (MVTR : Moisture Vapor Transmission Rate) supérieur à 300 g/m²/24 heures, de préférence supérieur ou égal à 600 g/m²/24 heures, de préférence encore supérieur ou égal à 1000 g/m²/24 heures.

Selon un autre mode de réalisation particulier, l'invention concerne un article adhésif sur la peau caractérisé en ce que le substrat **S** est un film souple continu en polyuréthane qui comporte une face de dessus **S1** enduit avec le gel silicone **G** selon l'invention et tel que défini ci-dessus et une face de dessous **S2** sur laquelle est aposé un adhésif sensible à la pression. Selon une variante avantageuse de l'invention le film souple continu en polyuréthane est perforé de manière à pouvoir favoriser la circulation des exsudats,

Ainsi l'article adhésif selon l'invention est selon un mode de réalisation particulier un stratifié adhésif amovible et présente l'avantage de pouvoir être utilisé en tant que couche de contact avec la peau dans différents types de pansements.

L'adhésif sensible à la pression peut être n'importe lequel des nombreux adhésifs sensibles à la pression connus de l'art. Ces adhésifs, généralement sous une forme anhydre et sans solvant, sont collants de façon permanente à température ambiante et adhèrent fermement à une variété de surfaces dissemblables lors d'un contact simple, sans qu'il y ait besoin d'utiliser plus que la pression d'un doigt ou de la main. Ils ne nécessitent pas d'activation par de l'eau, un solvant ou de la chaleur afin d'avoir une forte force adhésive de maintien. Des exemples d'adhésifs sensibles à la pression comprennent les adhésifs de caoutchouc/résine, qui sont des mélanges de matière caoutchouteuse et de résine dure, et les adhésifs acryliques (ou d'acrylate). La classe actuellement préférée d'adhésifs sensibles à la pression pour une utilisation dans la présente invention est celle des adhésifs acryliques.

Un autre objet de l'invention concerne donc un pansement ou patch à usage médical ou paramédical comprenant un article adhésif sur la peau selon l'invention et tel que décrit ci-dessus.

De préférence, les quantités de la composition silicone **A** seront déterminées de manière à obtenir des enductions ayant une teneur en gel silicone **G** comprise entre 20 et 500 g/m² de support, de préférence entre 40 et 350 g/m² et encore plus préférentiellement entre 80 et 250 g/m².

Comme technique pour déposer la composition silicone **A,** on peut citer par exemple les techniques d'enduction réalisées par racle, en particulier par racle sur cylindre, racle en l'air et racle sur tapis, ou par foulardage, c'est-à-dire par exprimage entre deux rouleaux, ou encore par rouleau lécheur, cadre rotatif, rouleau inverse "reverse roll", transfert, pulvérisation.

Comme autre technique d'enduction on peut citer la technique de couchage au rideau. Le couchage au rideau est un procédé d'application d'un liquide de couchage sur un article ou un support. Le couchage au rideau est caractérisé par la formation d'un rideau tombant librement d'un liquide de couchage qui tombe de la lèvre du Hopper et, sous l'effet de la gravité, vient rencontrer l'article se déplaçant à travers le rideau pour former un couchage (ou une enduction). Cette technique a été très utilisée dans le domaine de la préparation de supports argentiques photosensibles multicouches (voir par exemple les brevets US-3508947, US3508947 ou EP537086).

Il est connu que la qualité du couchage dépend de la qualité du rideau tombant librement, Il est préférable que le rideau présente un écoulement laminaire stable depuis l'endroit où il se forme jusqu'à la ligne rencontre avec le support en mouvement. A défaut, la tension superficielle amènera le rideau à se contracter vers l'intérieur et à interrompre l'écoulement laminaire. Afin d'éviter ce problème, il est connu d'employer des guides de bord pour saisir le rideau tombant librement au niveau de ses bords et empêcher sa contraction vers l'intérieur en raison de la tension superficielle. Des exemples de tels systèmes sont décrits dans les brevets US4933215, US4479987, US4974533, US3632374, US4479987, EP537086 et US4830887.

Un autre objet de l'invention concerne une prothèse mammaire comprenant une poche en polyuréthanne contenant le gel silicone **G** selon l'invention et tel que défini ci-dessus.

Le dernier objet de l'invention concerne un coussin ou matelas de prévention des escarres comprenant le gel silicone **G** selon l'invention et tel que défini ci-dessus.

Les exemples non limitatifs qui suivent, montrent diverses possibilités de formulation des compositions selon l'invention ainsi que les caractéristiques et les propriétés des gels silicones obtenus par réticulation desdites compositions.

### EXEMPLES

### 1) Mesure du collant instantané ou « tack » :

Le test est réalisé selon la norme ASTM D2979 avec un appareil PROBE TACK Device (PT-1000). Un poinçon cylindrique à face plane est amené en contact avec le gel du composite à tester (surface de contact avec le gel= 0.2cm²). Le composite est constitué d'un film souple en polyuréthane (supporté papier) enduit à 200 g/m² de la composition silicone précurseur du gel, Le poinçon est maintenu ensuite en contact avec le gel pendant un temps de contact de 1 seconde à une pression constante de 100 gf/cm². Ensuite, le poinçon est décollé à vitesse constante de 10 mm/s du gel, et l'énergie de décollement nécessaire pour séparer le gel de la tige est mesurée et s'exprime en mJ/cm².

### 2) Evaluation de l'adhésion (« Rub-Off ») du gel silicone au support qui est un film souple en polyuréthane et de la qualité du gel

L'adhésion du gel silicone au support qui est un film souple en polyuréthane ou mesure du « Rub-Off » consiste en une évaluation qualitative de la résistance du gel lors de gommages au doigt. La surface du gel est frottée au doigt et le nombre de passages (aller-retour) au doigt est compté jusqu'à l'apparition d'une délamination. Le gel sera considéré comme plus adhérent au film polyuréthane si le nombre de passages au doigt est plus important avant d'observer la délamination du gel de son substrat. Au-delà de 30 « gommages » aller-retour au doigt, on considère que l'adhésion au support en polyuréthane est satisfaisante.

La qualité du gel est évaluée par pénétrométrie (PEN) selon la norme NF ISO 2137, à l'aide d'un pénétromètre modèle PNR 12 Petrotest avec un poids total de la tige et du cône fixé à 62,5 g. La pénétrabilité au cône d'un gel silicone est déterminée à 25 °C en mesurant la profondeur de pénétration du cône dans l'échantillon, celle-ci étant obtenue en libérant l'ensemble cône du pénétromètre et en laissant le cône agir pendant 5 secondes. Les résultats sont exprimés en dixièmes de mm (mm/10).

### 3) Préparation des compositions silicones précurseurs de gel selon l'invention

### a) Matières premières utilisées

- POS **B1** = huile polydiméthylsiloxane α, ω(diméthylvinylsiloxy) ayant une viscosité dynamique à 25°C égale à 60000 mPa.s.
- POS **B2** = huile polydiméthylsiloxane α, ω(diméthylvinylsiloxy) ayant une viscosité dynamique à 25°C égale à 1000 mPa.s.
- POS **CE** = huile poly(diméthylsiloxy)-α, ω diméthylhydrogénosiloxy de viscosité d'environ 8,5 mPa.s , contenant en moyenne 5,7% en poids de motif SiH , ayant une structure du type M^{H}DₓM^{H} avec x compris en moyenne entre 7 et 15 et ayant un ratio (nombre de groupe SiH)/ (nombre d'atome de silicium total) = 0,154 ;
- POS XL¹ = huile poly(diméthylsiloxy) (méthylhydrogénosiloxy) α, ω diméthylhydrogénosiloxy de viscosité moyenne de 22 mPa.s, contenant 20,00 % en poids de groupement SiH (ou 0,694 % en poids d'atome d'hydrogène provenant de fonction SiH) par polymère et ayant une structure du type M^{H}DₓD_{w}^{H} M^{H} avec x compris en moyenne entre 18 et 20 et w en moyenne entre 15 et 17 et ayant un ratio (nombre de groupe SiH)/ (nombre d'atome de silicium total) = 0,478;
- POS **XL²** = huile poly(diméthylsiloxy) (méthylhydrogénosiloxy) α, ω diméthylhydrogénosiloxy de viscosité moyenne de 40 mPa.s, contenant 30,50 % en poids de groupement SiH (ou 1,059 % en poids d'atome d'hydrogène provenant de fonction SiH) par polymère et ayant une structure du type M^{H}DₓD_{w}^{H} M^{H} avec x compris en moyenne entre 17 et 19 et w en moyenne entre 36 et 38 et ayant un ratio (nombre de groupe SiH)/ (nombre d'atome de silicium total) = 0,655;
- Catalyseur d'hydrosilylation **E** (Cata E) = complexe organométallique du platine (Platine de Karstedt) utilisé comme catalyseur de la réaction d'hydrosilylation correspondant à 10% de platine.
- Inhibiteur **D** = inhibiteur de la réaction d'hydrosilylation = 1-éthynyl-1-cyclohexanol (ECH)

### b) Préparation des composites (=support enduit d'un gel silicone)

Les compositions silicones testées se présentent sous la forme bicomposante. Les parties nommées Partie A et Partie B sont mélangées ensuite dans un rapport en poids de 1:1. La composition avant réticulation est indiquée dans le tableau correspondant à l'essai. Puis on applique la composition silicone précurseur d'un gel à une teneur de 200 g/m² sur un support en polyuréthane (film souple supporté papier) à l'aide d'une racle d'enduction. Après l'enduction, on effectue la réticulation du composite pendant 30 min à 120°C en étuve ventilée de manière à obtenir un support enduit par un gel. Les résultats sont consignés dans les Tableaux 1 et 2 ci-dessous.

Dans ces tableaux, le symbole « x » signifie que la mesure n'a pas été effectuée car il a été obtenu un élastomère silicone « dur » c'est-à-dire distinct d'un « gel silicone ».

**Tableau 1. Propriétés des gels obtenus à partir d'une composition comprenant un réticulant POS XL¹ (« crosslinker ») contenant 20,00 % en poids de groupement SiH par polymère.**

| N° d'essai | C-1 | C-2 | C-3 | C-4 | C-5 | I-6 | I-7 |
|---|---|---|---|---|---|---|---|
| Parties en poids (pour 100 parties de la composition) | | | | | | | |
| POS B1 | 93,04 | 92,54 | 90,26 | 89,56 | 88,08 | 87,38 | 84,55 |
| POS B2 | 4,45 | 4,45 | 4,47 | 4,47 | 4,45 | 4,45 | 4,45 |
| ECH | 0,0060 | 0,0060 | 0,0060 | 0,0060 | 0,0060 | 0,0060 | 0,0060 |
| POS CE | 2,00 | 2,00 | 5,00 | 4,95 | 7,00 | 8,00 | 10,74 |
| POS XL¹ | 0,50 | 1,00 | 0,25 | 1,00 | 0,45 | 0,15 | 0,25 |
| Catalyseur Pt | 0,0055 | 0,0055 | 0,0055 | 0,0055 | 0,0055 | 0,0055 | 0,0055 |

| Calculs ratios des compositions silicones précurseurs d'un gel | | | | | | | |
|---|---|---|---|---|---|---|---|
| RHAlk | 2,3 | 3,5 | 3,5 | 5,3 | 5,3 | 5,2 | 7,3 |
| RH^{CE}V | 1,15 | 1,16 | 2,95 | 2,94 | 4,22 | 4,86 | 6,71 |
| RH^{CE} | 50,12 | 33,44 | 83,40 | 55,42 | 79,62 | 93,05 | 91,53 |

| Propriétés des gels obtenus après réticulation | | | | | | | |
|---|---|---|---|---|---|---|---|
| Etat physique = gel | Non | Non | Oui | Non | Non | Oui | Oui |
| Pénétrométrie (mm/10) | x | x | 124 | 23 | 63 | 270 | 189 |
| Tack (mJ/cm²) | x | x | 11 | <5 | 11 | 25 | 25 |
| Rub off nombre de passages | x | x | >50 | x | >50 | 25 | 25 |
| Gel adhérent sur support polyuréthane et ayant un bon tack (> 14 mJ/cm²) | Non | Non | Non | Non | Non | Oui | Oui |

**Tableau 2. Propriétés des gels obtenus à partir d'une composition comprenant un réticulant POS XL² (« crosslinker ») contenant 30,5 % en poids de groupement SiH par polymère.**

| N° d'essai | C-8 | C-9 | C-10 | C-11 | C-12 | C-13 | C-14 | C-15 | I-16 | | C-17 | I-18 | I-19 |
|---|---|---|---|---|---|---|---|---|---|---|---|---|---|
| Parties en poids (pour 100 parties de la composition) | | | | | | | | | | | | | |
| POS B1 | 94,79 | 93,29 | 91,14 | 91,54 | 90,69 | 88,29 | 87,54 | 85,29 | 77,28 | | 79,54 | 74,78 | 70,44 |
| POS 82 | 4,449 | 4,451 | 4,449 | 4,450 | 4,451 | 4,450 | 4,450 | 4,450 | 4,451 | | 4,450 | 4,451 | 4,438 |
| ECH | 0,006 | 0,006 | 0,006 | 0,006 | 0,006 | 0,006 | 0,006 | 0,006 | 0,006 | | 0,006 | 0,006 | 0,006 |
| POS CE | 0,50 | 2,00 | 4,25 | 3.75 | 4,75 | 7,00 | 7,50 | 10,00 | 18,01 | | 15,00 | 20,51 | 24,86 |
| POS XL² | 0,25 | 0,25 | 0,15 | 0,25 | 0,10 | 0,25 | 0,50 | 0,25 | 0,25 | | 1,00 | 0,25 | |
| Catalyseur Pt | 0,0055 | 0,0055 | 0,0055 | 0,0055 | 0,0055 | 0,0055 | 0,0055 | 0,0055 | 0,0055 | | 0,0055 | 0,0055 | 0,0055 |

| Calculs ratios des compositions silicones précurseurs d'un gel | | | | | | | | | | | | | |
|---|---|---|---|---|---|---|---|---|---|---|---|---|---|
| RHAlk | 1,1 | 1,9 | 12,9 | 3,0 | 3,1 | 5,0 | 6,2 | 7,0 | 13,0 | | 13,4 | 15,1 | 19.0 |
| RH^{CE}V | 0,28 | 1,14 | 2,46 | 2,16 | 2,76 | 4,17 | 4,50 | 6,14 | 12,05 | | 9,79 | 14,11 | 18,02 |
| RH^{CE} | 26,47 | 59,02 | 83,61 | 72,97 | 89,53 | 83,44 | 72,97 | 87,80 | 92,84 | | 72,97 | 93,65 | 94,74 |

| Propriétés des gels obtenus après réticulation | | | | | | | | | | | | | |
|---|---|---|---|---|---|---|---|---|---|---|---|---|---|
| Etat physique = gel | Non | Non | Oui | Non | oui | Non | Non | Non | Oui | | Non | Oui | Oui |
| Pénétrométrie (mm/10) | x | x | 139 | 64 | 224 | x | x | x | 95 | | x | 118 | 125 |
| Tack (mJ/om²) | x | x | 13 | 11 | 13 | x | x | x | 14 | | x | 15 | 15 |
| Rub off nombre de passages | x | x | x | >50 | >50 | x | x | x | >50 | | x | >50 | >50 |
| Gel adhérent sur support polyuréthane et ayant un bon tack (> 14 mJ/cm²) | Non | Non | Non | Non | Non | Non | Non | Non | Oui | | Non | Oui | Oui |

## Revendications

1. Composition silicone **A** précurseur d'un gel silicone **G** et réticulable par hydrosilylation comprenant :
1) au moins un organopolysiloxane **B** comprenant:
(i) au moins deux motifs siloxyles de formule **(B1)** :
(Y)ₐ(Z)_{b}SiO_{(4-(a+b)/2} **(B1)**
dans laquelle :
- Y représente un radical monovalent contenant de 2 à 6 atomes de carbone, ayant au moins un groupe alcényle,
- Z représente un radical monovalent contenant de 1 à 20 atomes de carbone et ne comprenant pas de groupe alcényle;
- a et b représentent des nombres entiers, a valant 1, 2 ou 3, b valant 0, 1 ou 2 et (a+b) valant 1, 2 ou 3 ;
(ii) et comportant éventuellement d'autres motifs siloxyles de formule **(B2)** :
(Z)_{c}SiO_{(4-c)/2} **(B2)**
dans laquelle :
- Z a la même signification que ci-dessus, et
- c représente un nombre entier valant 0, 1, 2 ou 3,
2) au moins un organopolysiloxane **CE** comprenant :
- deux motifs siloxyles terminaux, identiques ou différents, de formule **(CE-1):**
(H)ₚ(R¹)_{q}SiO_{1/2} **(CE-1)**
dans laquelle :
- le symbole R¹ correspond à un groupe alkyle en C₁ à C₈ ou un groupe aryle en C₆ à C₁₀ ;
- et le symbole H représente un atome d'hydrogène, avec p= 0 ou 1, q= 2 ou 3 et (p+q)= 3;
- au moins un motif siloxyle de formule **(CE-2)** :
(H)ₙ(R²)ₘSiO_{2/2} **(CE-2)**
dans laquelle le radical R² correspond à un groupe alkyle en C₁ à C₈ ou un groupe aryle en C₆ à C₁₀, le symbole H représente un atome d'hydrogène, et n= 0 ou 1, m =1 ou 2 et (n+m)= 2, et
- avec la condition selon laquelle l'organopolysiloxane **CE** contient deux atomes d'hydrogène liés chacun à un atome de silicium différent par polymère, et de préférence l'organopolysiloxane **CE** contient par polymère deux motifs siloxyles de formule **(CE-1)** dans laquelle p=1 et au moins un motif siloxyle de formule **(CE-2)** dans laquelle n=0 ;
3) au moins un organopolysiloxane **XL** comprenant :
• au moins trois motifs siloxyles de formule **(XL-1):**
(H) (L)ₑ SiO_{(3-e)/2} **(XL-1)**
dans laquelle le symbole H représente un atome d'hydrogène, le symbole L représente un alkyle ayant de 1 à 8 atomes de carbone inclus ou un aryle en C₆ à C₁₀, et le symbole e est égal à 0, 1 ou 2 ; et
• éventuellement des autres motifs siloxyles de formule **(XL-2):**
(L)_{g} SiO_{(4-g)/2} **(XL-2)**
dans laquelle le symbole L représente un alkyle ayant de 1 à 8 atomes de carbone inclus ou un aryle en C₆ à C₁₀ et le symbole g est égal à 0, 1, 2 ou 3, et
• avec la condition selon laquelle l'organopolysiloxane **XL** contient entre 15,1 % et 40,0 % en poids de fonction Si-H par polymère, et de préférence entre 18 % et 35,0 % en poids de fonction Si-H par polymère,
4) une quantité efficace d'au moins un catalyseur d'hydrosilylation **E,**
5) au moins un inhibiteur **D** de la réaction d'hydrosilylation,
6) éventuellement au moins un additif **K,** et
les quantités en poids des organopolysiloxanes **B, CE** et **XL** sont choisies de manière à satisfaire les trois conditions suivantes :
a) le rapport molaire RHAlk = tH/tAlk ≥ 3,0 de préférence 3,0 ≤ RHAlk ≤24 et encore plus préférentiellement entre 4,5 ≤ RHAlk ≤ 20;
b) le rapport molaire RH^{CE}V = nH^{CE}/tAlk ≥ 4,50, et de préférence 4,50 ≤ RH^{CE}V ≤20, et
c) le % molaire RH^{CE} = (nH^{CE}/tH) x 100 est supérieur ou égal à 90 % molaire, et de préférence compris entre 90% molaire et 95% molaire, avec :
- tH = nombre de moles d'atome d'hydrogène directement lié à un atome de silicium des organopolysiloxanes **CE** et **XL,**
- tAlk = nombre de mole d'alcényle directement lié à un atome de silicium de l'organopolysiloxane **B** ; et
- nH^{CE} = nombre de mole d'atome d'hydrogène directement lié à un atome de silicium de l'organopolysiloxane **CE.**

2. Composition silicone **A** selon la revendication 1 dans laquelle :
- l'organopolysiloxane **B** à une viscosité dynamique à 25°C comprise entre 100 mPa.s et 120000 mPa.s,
- l'organopolysiloxane **CE** à une viscosité dynamique à 25°C comprise entre 1 mPa.s et 500 mPa.s, et de préférence entre 5 et 200 mPa.s, et
- l'organopolysiloxane **XL** à une viscosité dynamique à 25°C comprise entre 5 mPa.s et 2000 mPa.s, et de préférence entre 5 et 500 mPa.s., viscosités étant mesurées selon la méthode décrite dans la description.

3. Composition silicone **A** selon la revendication 2 dans laquelle la nature et les quantités en poids des organopolysiloxanes **B, CE** et **XL** sont choisis de manière à ce que la viscosité dynamique à 25°C de la composition silicone **A** soit comprise entre 200 mPa.s et 100000 mPa.s, et de préférence comprise entre 200 mPa.s et 80000 mPa.s., viscosités étant mesurées selon la méthode décrite dans la description.

4. Composition silicone **A** selon la revendication 1 **caractérisée en ce qu'**elle comprend au moins deux organopolysiloxanes **B** comportant, par molécule, au moins deux radicaux alcényles en C₂ à C₆ liés chacun à un atome de silicium, le premier ayant une viscosité dynamique à 25°C comprise entre 50000 mPa.s et 120000 mPa.s, et le deuxième ayant une viscosité dynamique à 25°C comprise entre 500 mPa.s et 20000 mPa.s., viscosités étant mesurées selon la méthode décrite dans la description.

5. Gel silicone **G** obtenu par réticulation de la composition **A** telle que définie selon l'une quelconque des revendications 1 à 4, de préférence par chauffage à une température comprise entre 70°C et 200°C et/ou sous l'action d'un rayonnement infrarouge.

6. Utilisation du gel silicone **G** tel que défini selon la revendication 5 dans les dispositifs médicaux tels qu'une prothèse mammaire, un pansement adhésif à la peau ou un dispositif de maintien d'accessoires médicaux utilisés au contact de la peau de type capteur, sonde, cathéter ou aiguille.

7. Article adhésif sur la peau comprenant un substrat **S** qui est un film en plastique, de préférence un film en polyester ou en polyuréthane, enduit de manière continue ou discontinue sur au moins une des deux faces par un gel silicone **G** selon la revendication 5.

8. Article adhésif sur la peau selon la revendication 7 dans lequel le substrat **S** est un film souple perforé en polyuréthane ou un film souple continu en polyuréthane.

9. Pansement ou patch à usage médical ou paramédical comprenant un article adhésif sur la peau tel que décrit selon la revendication 7 ou 8.

10. Prothèse mammaire comprenant une poche en polyuréthanne contenant le gel silicone **G** tel que défini selon la revendication 5.

11. Coussins ou matelas de prévention des escarres comprenant le gel silicone **G** tel que défini selon la revendication 5.

## Patentansprüche

1. Silikonzusammensetzung A, die eine Vorstufe eines Silikongels G darstellt und mittels Hydrosilylierung vernetzt werden kann, wobei sie Folgendes umfasst:
1) mindestens ein Organopolysiloxan B, das Folgendes umfasst:
(i) mindestens zwei Siloxylbausteine der Formel (B1) :
(Y)ₐ(Z)_{b}SiO_{(4-(a+b)/2} (B1)
wobei:
- Y für einen einbindigen Rest steht, der 2 bis 6 Kohlenstoffatome enthält, wobei er mindestens eine Alkenylgruppe hat,
- Z für einen einbindigen Rest steht, der 1 bis 20 Kohlenstoffatome enthält, wobei er keinerlei Alkenylgruppe umfasst;
- a und b für ganze Zahlen stehen, wobei a die Werte 1, 2 oder 3 annimmt, b die Werte 0, 1 oder 2 annimmt und (a+b) die Werte 1, 2 oder 3 annimmt;
(ii) und das möglicherweise weitere Siloxylbausteine der Formel (B2) aufweist:
(Z)_{c}SiO_{(4-c)/2} (B2)
wobei:
- Z dieselbe Bedeutung wie oben hat, und
- c für eine ganze Zahl steht, welche die Werte 0, 1, 2 oder 3 annimmt,
2) mindestens ein Organopolysiloxan CE, das Folgendes umfasst:
- zwei endständige Siloxylbausteine, die gleichartig oder verschiedenartig sind, der Formel (CE-1):
(H)ₚ(R¹)qSiO_{1/2} (CE-1)
wobei:
- das Zeichen R¹ einer C₁-C₈-Alkylgruppe oder einer C₆-C₁₀-Arylgruppe entspricht;
- und das Zeichen H für ein Wasserstoffatom steht, mit p = 0 oder 1, q = 2 oder 3 und (p+q) = 3;
- mindestens einen Siloxylbaustein der Formel (CE-2) :
(H)ₙ(R²)ₘSiO_{2/2} (CE-2)
wobei der Rest R² einer C₁-C₈-Alkylgruppe oder einer C₆-C₁₀-Arylgruppe entspricht, wobei das Zeichen H für ein Wasserstoffatom steht, und n = 0 oder 1 ist, m = 1 oder 2 ist und (n+m) = 2 ist, und
- mit der Maßgabe, dass das Organopolysiloxan CE pro Polymer zwei Wasserstoffatome enthält, die jeweils an ein unterschiedliches Siliciumatom gebunden sind, wobei das Organopolysiloxan CE vorzugsweise pro Polymer zwei Siloxylbausteine der Formel (CE-1), in welcher p = 1 ist, und mindestens einen Siloxylbaustein der Formel (CE-2) enthält, in welcher n = 0 ist;
3) mindestens ein Organopolysiloxan XL, das Folgendes umfasst:
• mindestens drei Siloxylbausteine der Formel (XL-1) :
(H) (L)ₑ SiO_{(3-e)/2} (XL-1)
wobei das Zeichen H für ein Wasserstoffatom steht, das Zeichen L für ein Alkyl mit 1 bis 8 Kohlenstoffatomen einschließlich der Grenzwerte oder ein C₆-C₁₀-Aryl steht und das Zeichen e gleich 0, 1 oder 2 ist; und
• möglicherweise weitere Siloxylbausteine der Formel (XL-2):
(L)_{g} SiO_{(4-g)/2} (XL-2)
wobei das Zeichen L für ein Alkyl mit 1 bis 8 Kohlenstoffatomen einschließlich der Grenzwerte oder ein C₆-C₁₀-Aryl steht und das Zeichen g gleich 0, 1, 2 oder 3 ist, und
• mit der Maßgabe, dass das Organopolysiloxan XL zwischen 15,1 % und 40,0 % nach Gewicht an Si-H-Funktionen pro Polymer, und vorzugsweise zwischen 18 % und 35,0 % nach Gewicht an Si-H-Funktionen pro Polymer, enthält,
4) eine wirksame Menge mindestens eines Hydrosilylierungskatalysators E,
5) mindestens einen Hemmer D der Hydrosilylierungsreaktion,
6) möglicherweise mindestens einen Zusatzstoff K, und
wobei die gewichtsbezogenen Mengen an den Organopolysiloxanen B, CE und XL derart gewählt sind, dass die folgenden drei Maßgaben erfüllt werden:
a) das Molverhältnis RHAlk = tH/tAlk ist ≥ 3,0, wobei vorzugsweise 3,0 ≤ RHAlk ≤ 24 ist und es mit noch größerem Vorzug im Bereich von 4,5 ≤ RHAlk ≤ 20 liegt;
b) das Molverhältnis RH^{CE}V = nH^{CE}/tAlk ist ≥ 4,50, wobei vorzugsweise 4,50 ≤ RH^{CE}V ≤ 20 ist; und
c) der molare %-Wert RH^{CE} = (nH^{CE}/tH) x 100 ist größer oder gleich 90 Mol-% und liegt vorzugsweise zwischen 90 Mol-% und 95 Mol-%, mit:
- tH = Molanzahl an Wasserstoffatomen, die unmittelbar an ein Siliciumatom der Organopolysiloxane CE und XL gebunden sind,
- tAlk = Molanzahl an Alkenyl, das unmittelbar an ein Siliciumatom des Organopolysiloxans B gebunden ist; und
- nH^{CE} = Molanzahl an Wasserstoffatomen, die unmittelbar an ein Siliciumatom der Organopolysiloxane CE gebunden sind.

2. Silikonzusammensetzung A nach Anspruch 1, wobei:
- das Organopolysiloxan B bei 25 °C eine dynamische Viskosität hat, die im Bereich von 100 mPa.s bis 120.000 mPa.s liegt,
- das Organopolysiloxan CE bei 25 °C eine dynamische Viskosität hat, die im Bereich von 1 mPa.s bis 500 mPa.s, und vorzugsweise von 5 bis 200 mPa.s liegt, und
- das Organopolysiloxan XL bei 25 °C eine dynamische Viskosität hat, die im Bereich von 5 mPa.s bis 2.000 mPa.s, und vorzugsweise von 5 bis 500 mPa.s liegt; wobei die Viskositäten nach der Methode gemessen werden, welche in der Beschreibung dargelegt ist.

3. Silikonzusammensetzung A nach Anspruch 2, wobei die Beschaffenheit und die gewichtsbezogenen Mengen der Organopolysiloxane B, CE und XL derart gewählt sind, dass die dynamische Viskosität der Silikonzusammensetzung A bei 25 °C im Bereich von 200 mPa.s bis 100.000 mPa.s, und vorzugsweise im Bereich von 200 mPa.s bis 80.000 mPa.s liegt; wobei die Viskositäten nach der Methode gemessen werden, welche in der Beschreibung dargelegt ist.

4. Silikonzusammensetzung A nach Anspruch 1, **dadurch gekennzeichnet, dass** sie mindestens zwei Organopolysiloxane B umfasst, die pro Molekül mindestens zwei C₂-C₆-Alkenylreste aufweisen, welche jeweils an ein Siliciumatom gebunden sind, wobei das erste bei 25 °C eine dynamische Viskosität im Bereich von 50.000 mPa.s bis 120.000 mPa.s hat, und wobei das zweite bei 25 °C eine dynamische Viskosität im Bereich von 500 mPa.s bis 20.000 mPa.s hat; wobei die Viskositäten nach der Methode gemessen werden, welche in der Beschreibung dargelegt ist.

5. Silikongel G, das erhalten wird, indem die Zusammensetzung A gemäß der Begriffsbestimmung in einem beliebigen der Ansprüche 1 bis 4 vernetzt wird, vorzugsweise durch Erhitzen auf eine Temperatur im Bereich von 70 °C bis 200 °C und/oder unter Einwirkung von Infrarotstrahlung.

6. Verwendung des Silikongels G gemäß der Begriffsbestimmung in Anspruch 5 in medizinischen Vorrichtungen wie etwa einem Brustimplantat, einem auf der Haut haftenden Pflaster oder einer Haltevorrichtung für medizinisches Zubehör, bei dessen Verwendung ein Hautkontakt hergestellt wird, vom Typ Sensor, Sonde, Katheter oder Nadel.

7. Gegenstand, der auf der Haut haftet, wobei er ein Substrat S umfasst, bei welchem es sich um eine Kunststofffolie, vorzugsweise um eine Folie aus Polyester oder aus Polyurethan handelt, welche durchgehend oder abschnittsweise auf mindestens einer der beiden Seiten mit einem Silikongel G nach Anspruch 5 überzogen ist.

8. Gegenstand, der auf der Haut haftet, nach Anspruch 7, wobei es sich bei dem Substrat S um eine weiche perforierte Folie aus Polyurethan oder um eine weiche durchgehende Folie aus Polyurethan handelt.

9. Verbandsmaterial oder Pflaster zur medizinischen oder paramedizinischen Verwendung, wobei es einen Gegenstand umfasst, welcher auf der Haut haftet und der Beschreibung in Anspruch 7 oder 8 entspricht.

10. Brustimplantat, das einen Beutel aus Polyurethan umfasst, welcher das Silikongel G gemäß der Begriffsbestimmung in Anspruch 5 enthält.

11. Kissen oder Matratzen, die zur Verhütung von Wundliegegeschwüren dienen und das Silikongel G gemäß der Begriffsbestimmung in Anspruch 5 umfassen.

## Claims

1. Silicone composition **A** which is a precursor of a silicone gel **G** and which is crosslinkable by hydrosilylation comprising:
1) at least one organopolysiloxane **B** comprising:
(i) at least two siloxyl units of formula **(B1) :**
(Y)ₐ(Z)_{b}SiO_{(4-(a+b)/2} **(B1)**
in which:
- Y represents a monovalent radical containing from 2 to 6 carbon atoms, having at least one alkenyl group,
- Z represents a monovalent radical containing from 1 to 20 carbon atoms and not comprising an alkenyl group;
- a and b represent integers, a being 1, 2 or 3, b being 0, 1 or 2 and (a+b) being 1, 2 or 3;
(ii) and optionally comprising other siloxyl units of formula **(B2):**
(Z)_{c}SiO_{(4-c)/2} **(B2)**
in which:
- Z has the same meaning as above, and
- c represents an integer which is 0, 1, 2 or 3,
2) at least one organopolysiloxane **CE** comprising:
- two siloxyl end units, which may be identical or different, of formula **(CE-1):**
(H)ₚ(R¹)_{q}SiO_{1/2} **(CE-1)**
in which:
- the symbol R¹ corresponds to a C₁ to C₈ alkyl group or to a C₆ to C₁₀ aryl group;
- and the symbol H represents a hydrogen atom, with p= 0 or 1, q= 2 or 3 and (p+q) = 3;
- at least one siloxyl unit of formula **(CE-2):**
(H)ₙ(R²)ₘSiO_{2/2} **(CE-2)**
in which the radical R² corresponds to a C₁ to C₈ alkyl group or a C₆ to C₁₀ aryl group, the symbol H represents a hydrogen atom and n= 0 or 1, m =1 or 2 and (n+m) = 2, and
- with the condition according to which the organopolysiloxane **CE** contains two hydrogen atoms each one bonded to a different silicon atom per polymer, and preferably the organopolysiloxane **CE** contains, per polymer, two siloxyl units of formula **(CE-1)** in which p=1 and at least one siloxyl unit of formula **(CE-2)** in which n=0 ;
3) at least one organopolysiloxane **XL** comprising:
• at least three siloxyl units of formula **(XL-1):**
(H) (L)ₑ SiO_{(3-e)/2} **(XL-1)**
in which the symbol H represents a hydrogen atom, the symbol L represents an alkyl having from 1 to 8 carbon atoms inclusive or a C₆ to C₁₀ aryl, and the symbol e is equal to 0, 1 or 2; and
• optionally other siloxyl units of formula **(XL-2):**
(L)_{g} SiO_{(4-g)/2} **(XL-2)**
in which the symbol L represents an alkyl having from 1 to 8 carbon atoms inclusive or a C₆ to C₁₀ aryl and the symbol g is equal to 0, 1, 2 or 3, and
• with the condition according to which the organopolysiloxane **XL** contains between 15.1% and 40.0% by weight of Si-H function per polymer, and preferably between 18% and 35.0% by weight of Si-H function per polymer,
4) an effective amount of at least one hydrosilylation catalyst **E,**
5) at least one hydrosilylation reaction inhibitor **D,**
6) optionally at least one additive **K,** and
the weight amounts of the organopolysiloxanes **B, CE** and **XL** are chosen so as to satisfy the following three conditions:
a) the molar ratio RHAlk = tH/tAlk ≥ 3.0, preferably 3.0 ≤ RHAlk ≤ 24 and even more preferentially between 4.5 ≤ RHAlk ≤ 20;
b) the molar ratio RH^{CE}V = nH^{CE}/tAlk ≥ 4.50 and preferably 4.50 ≤ RH^{CE}V ≤ 20, and
c) the mol% RH^{CE} = (nH^{CE}/tH) x 100 is greater than or equal to 90 mol%, and preferably between 90 mol% and 95 mol%, with:
- tH = number of moles of hydrogen atom directly bonded to a silicon atom of the organopolysiloxanes **CE** and **XL,**
- tAlk = number of moles of alkenyl directly bonded to a silicon atom of the organopolysiloxane **B;** and
- nH^{CE} = number of moles of hydrogen atom directly bonded to a silicon atom of the organopolysiloxane **CE.**

2. Silicone composition **A** according to Claim 1, wherein:
- the organopolysiloxane **B** has a dynamic viscosity at 25°C of between 100 mPa.s and 120 000 mPa.s,
- the organopolysiloxane **CE** has a dynamic viscosity at 25°C of between 1 mPa.s and 500 mPa.s, and preferably between 5 and 200 mPa.s, and
- the organopolysiloxane **XL** has a dynamic viscosity at 25°C of between 5 mPa.s and 2000 mPa.s, and preferably between 5 and 500 mPa.s, viscosities being measured according to the method described in the description.

3. Silicone composition **A** according to Claim 2, wherein the nature and the weight amounts of the organopolysiloxanes **B, CE** and **XL** are chosen such that the dynamic viscosity at 25°C of the silicone composition **A** is between 200 mPa.s and 100 000 mPa.s, and preferably between 200 mPa.s and 80 000 mPa.s, viscosities being measured according to the method described in the description.

4. Silicone composition **A** according to Claim 1, **characterized in that** it comprises at least two organopolysiloxanes **B** comprising, per molecule, at least two C₂ to C₆ alkenyl radicals each bonded to a silicon atom, the first having a dynamic viscosity at 25°C of between 50 000 mPa.s and 120 000 mPa.s, and the second having a dynamic viscosity at 25°C of between 500 mPa.s and 20 000 mPa.s, viscosities being measured according to the method described in the description.

5. Silicone gel **G** obtained by crosslinking of the composition **A** as defined in any one of Claims 1 to 4, preferably by heating at a temperature of between 70°C and 200°C and/or under the action of infrared radiation.

6. Use of the silicone gel **G** as defined in Claim 5, in medical devices such as a mammary prosthesis, an adhesive dressing which adheres to the skin or a device for holding in place medical accessories used in contact with the skin, of sensor, probe, catheter or needle type.

7. Item which adheres to the skin, comprising a substrate **S** which is a plastic film, preferably a polyester or polyurethane film, continuously or discontinuously coated onto at least one of the two faces with a silicone gel **G** according to Claim 5.

8. Item which adheres to the skin according to Claim 7, wherein the substrate **S** is a perforated flexible polyurethane film or a continuous flexible polyurethane film.

9. Dressing or patch for medical or paramedical use, comprising an item which adheres to the skin as described in Claim 7 or 8.

10. Mammary prosthesis comprising a polyurethane pouch containing the silicone gel **G** as defined in Claim 5.

11. Cushions or mattresses for preventing pressure sores, comprising the silicone gel **G** as defined in Claim 5.
